# EUROPÄISCHE PATENTSCHRIFT

(11) **EP 1 487 514 B1**
(45) Veröffentlichungstag und Bekanntmachung des Hinweises auf die Patenterteilung: **14.06.2006**
(21) Anmeldenummer: 03724834.1
(22) Anmeldetag: 17.03.2003
(51) Int. Cl.: A61L 31/16

(54) **ABSCHEIDUNGSVERFAHREN FÜR ENDOPROTHESEN ZUR GLEICHMÄSSIGEN MEDIKAMENTENABGABE**
DEPOSITION METHOD FOR ENDOPROSTHESES PROVIDED FOR CONSTANTLY ADMINISTERING MEDICAMENTS
PROCEDE DE PRECIPITATION PAR DEPOT POUR ENDOPROTHESES A DES FINS DE DISTRIBUTION MEDICAMENTEUSE HOMOGENE

(30) Priorität: 16.03.2002 DE 20204258 U
(43) Veröffentlichungstag der Anmeldung: 22.12.2004
(73) Patentinhaber: Aachen Resonance Entwicklungs-Gesellschaft für Magnetresonanz - Kompatible Medizinprodukte mbH, 42074 Aachen (DE)
(72) Erfinder: KLEE, Doris, 52074 Aachen (DE); WEISS, Norbert, 52525 Heinsberg (DE); RÜBBEN, Alexander, 52074 Aachen (DE); BÜCKER, Arno, 52349 Düren (DE)
(74) Vertreter: Theobald, Andreas
(86) Internationale Anmeldenummer: PCT/DE2003/000848
(87) Internationale Veröffentlichungsnummer: WO 2003/077967

(56) Entgegenhaltungen:
- WO-A-01/49268
- WO-A-02/085337
- DE-A- 19 604 173
- US-B1- 6 231 600

## Beschreibung

Die Erfindung betrifft ein Verfahren zur Erzeugung von Endoprothesen (z.B. Stents) mit gleichmäßiger Wirkstoffabgabe, die durch die Löslichkeit des Wirkstoffs im Gewebe bedingt ist. Die Stents werden zunächst mit einer funktionellen Polymerschicht versehen, um die Beladungsmenge mit dem Wirkstoff zu erhöhen und zusammen mit dem beschriebenen Abscheidungsverfahren eine gleichmäßige Wirkstoffabgabe zu erreichen.

Die sogenannten "minimalinvasiven Verfahren" nehmen in der Medizin einen immer größeren Stellenwert ein. Im Rahmen der Radiologie ist hierbei die interventionelle Radiologie anzusprechen, die wesentlich zur Entwicklung minimalinvasiver Techniken und hierfür notwendiger Geräte und Prothesen aus geeigneten Materialien beigetragen hat. So werden heute kleine Metallgitter als Gefäßendoprothesen, sog. Stents, sowohl von Kardiologen als auch von Radiologen in Gefäße eingesetzt, um diese offen zu halten. Bei herkömmlichen Stents kommt es jedoch häufig zu einer Verdickung der Gefäßwand mit konsekutiver Lumeneinengung im Bereich des Stents durch eine Zellproliferation oder durch eine Anlagerung von Zellen.

Durch Medikamentenabgabe von der Stentoberfläche, die zur Verbesserung der Medikamentenbeladung und Medikamentenabgabe strukturiert und/oder mit einer geeigneten polymeren Beschichtung versehen sein kann, kann diesem Problem entgegengewirkt werden. Dies wird zumindest von ersten Studien, die allerdings noch keine Langzeiterfahrungen über mehrere Jahre beinhalten, nahegelegt (Sousa JE, et al. Sirolimus-eluting stent for the treatment of in-stent restenosis: a quantitative coronary angiography and three-dimensional intravascular ultrasound study. Circulation, 2003; 107: 24-27).

Zur Herstellung der beschichteten Endoprothesen wurden verschiedene Verfahren vorgeschlagen. Als Stand der Technik kann das Aufbringen einer Polymerbeschichtung und die anschließende Bindung eines Wirkstoffes mittels verschiedener Verfahren an das Polymer angesehen werden. Um eine ausreichend langsame Abgabe des Wirkstoffes zu erreichen, sind bisher komplizierte Verfahren notwendig, bei denen z.B. auf eine nicht poröse erste Polymerbeschichtung zunächst der Wirkstoff und sodann eine zweite poröse Polymerbeschichtung zur Verhinderung einer zu schnellen Wirkstoffabgabe erfolgt.

Die WO 01/049 268 A1 beschreibt eine pharmazeutische Rezeptur, in welcher ein schwer wasserlöslicher Wirkstoff in eine Matrix aus einem hydrophilen Polymer eingebaut wird, welche geschluckt wird oder unter Umgehung des Verdauungstraktes verabreicht wird. Im menschlichen Körper kann sich die Matrix auflösen und so den Wirkstoff freisetzen.

Der Erfindung liegt die Aufgabe zugrunde, ein Verfahren der eingangs genannten Art derart weiter zu entwickeln, dass es den gezielten Aufbau einer funktionellen Polymerschicht gestattet, die anschließend mit einer weiteren biologisch aktiven Beschichtung, d.h. mit einer Schicht nicht kovalent gebundener schwer wasserlöslicher Wirkstoffmoleküle, versehen wird. Die Wirkstoffabgabe wird hierbei nicht durch das Polymer beeinflusst, sondern hängt im wesentlichen von der Löslichkeit der Wirkstoffmoleküle im Gewebe ab. Bei den abzuscheidenden Wirkstoffen handelt es sich um im wesentlichen wasserunlösliche (Löslichkeit von weniger als 0,1 mg/ml in destilliertem Wasser bei 25°C) oder in Wasser nur schwer lösliche Substanzen (Löslichkeit von 0,1 bis 0,9 mg/ml destilliertem Wasser), wie z.B. Tretinoin und Tretinoinderivate, Orphanrezeptoragonisten, Elafinderivate, Corticosteroide und Steroidhormone (wie z.B. Methylprednisolon, Dexamethason, Östradiol), Taxol, Taxolderivate, Rapamune, Tacrolimus, hydrophobe Proteine oder zellproliferationsverändernde Substanzen. Die Beschichtung ist erfindungsgemäß auf Gegenstände unterschiedlicher Materialbeschaffenheit anwendbar, wie z.B. Metallen, Polymeren, Keramiken. Als Endoprothesen kommen z.B. in Betracht: Stents, Stent-grafts, Gefäßclips, Filter, Verschlusssysteme, ummantelte Stents.

Die Aufgabe der Beschichtung mit gleichmäßiger Wirkstoffabgabe wird erfindungsgemäß bei einem Verfahren der eingangs genannten Art dadurch gelöst, dass zunächst zur Herstellung der Polymerschicht aus den Ausgangsverbindungen der allgemeinen Strukturen (1), (2) und (3) bei erhöhten Temperaturen und reduzierten Drücken im wesentlichen Monomere in der Gasphase erzeugt werden und diese anschließend durch Abkühlung spontan polymerisiert werden, mit:

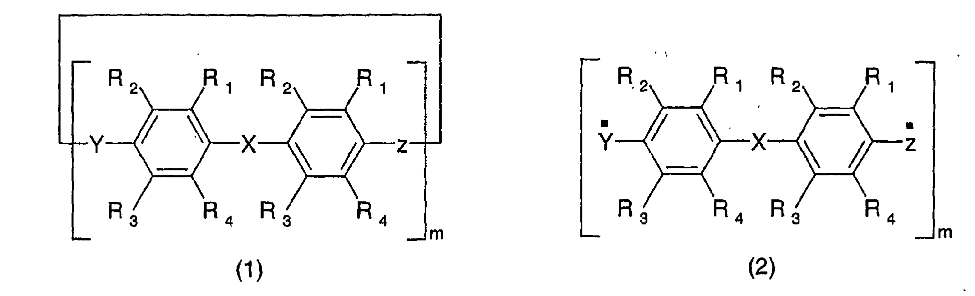

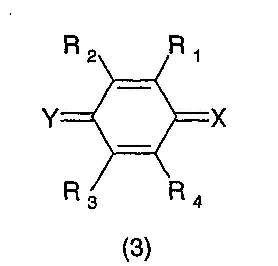

R_{1,2,3,4}: jeweils, gleich oder verschieden voneinander, Wasserstoffatome, Halogenatome, Alkylgruppen bzw. substituierte Alkylgruppen, Arylgruppen bzw. substituierte Arylgruppen, organische Reste oder Radikale, Gruppen der allgemeinen Struktur CO(O-M-A) (mit M: aliphatische oder aromatische Gruppen und A: z.B. Wasserstoff, Hydroxyl-, Amino-, Carboxylgruppen), metallierte Gruppen, Hydroxylgruppen, Aminogruppen, Carboxylgruppen, Estergruppen, Ethergruppen, Säurehalogenidgruppen, Isocyanatgruppen, schwefelhaltige Gruppen (z.B. Sulfonsäure-, Thioether-, Schwefelsäuregruppen), stickstoffhaltige Gruppen (z.B. Nitril-, Amid-, Nitro-, Nitrosamingruppen), phosphorhaltige Gruppen (z.B. Phosphorsäureester-, Phosphonatgruppen), siliziumhaltige Gruppen (z.B. Silyl-, Silyloxygruppen)
X, Y: Kohlenwasserstoffreste: z.B.: Methylen-, Isopropyliden-, Ethylengruppen, funktionalisierte Kohlenwasserstoffreste
m: Zahl der Wiederholungseinheiten = 1-20, bevorzugt 2-10, ganz bevorzugt 2-5

Bevorzugt sind folgende Gruppen für R1-R4: Wasserstoff, Halogen, C1-C6 Alkyl, C1-C3-Alkylthio-, C6-C12-Aryl-, Nitro-, Carbamoyl-, C1-C4 Alkoxy, -CN, CF₃, NH₂ -, Carboxy-, C1-C4 Alkoxycarbonyl, C1-C4 N-alkyl-carbamoyl oder C1-C5 Alkenyl

Die als Reste R1, R2, R3 oder R4 genannten Alkyl-, Alkoxy-, Alkylthio-, Alkoxycarbonyl-, N-alkyl-carbamoyl-, Alkenyl-, Alkylcarboxy- oder Alkylsulfonyl-Gruppen können verzeigt oder unverzeigt bzw. offenkettig oder cyclisch sein. Desweiteren können sie vorzugsweise Substituenten, wie z.B. Halogenatome, Cyano-, Carboxy-, Carbonyl-, Nitril-, Carbamoyl-, C1-C4-Alkoxy, Phenyl, C1-C4 Alkoxycarbonyl-, C1-C4 Alkylcarboxy-, C1-C4 N-Alkyl-carbamoyl-, C1-C4 N-Dialkylcarbamoyl-, Hydroxy-, Nitro.-, SO₃H-, Ether-, Sulfamoyl-, C1-C4 N-Alkylsulfamoyl, C1-C4 Dialkylsulfamoyl, -CO-R (mit: R = OH, O-Alkyl, NH-Alkyl), Trifluormethyl-Gruppierungen oder weitere offenkettige oder cyclische Gruppierungen mit Heteroatomen (z.B. mit N, S und/oder O) aufweisen, bei denen ggf. die Heteroatome Teil eines Heterocyclus sein können. Das mögliche Vorhandensein von vorgenannten Substituenten gilt für alle als Reste R1, R2, R3 oder R4 genannten Gruppen (soweit substituierbar), insbesondere für die dort genannten Alkyl-, Cycloalkyl-, Alkenyl-, Cycloalkyl- und C6-C12 Aryl-Gruppen.

Unter "Halogen" wird Fluor, Chlor, Brom oder Iod verstanden.

Unter C1-C4 Alkyl sind Methyl, Ethyl., n-Propyl-, Isopropyl-, n-Butyl, Isobutyl., sek. Butyl. und tert-Butyl zu verstehen.

Unter C1-C8 Alkyl sind u.a. Methyl, Ethyl., n-Propyl-, Isopropyl-, n-Butyl, Isobutyl., sek. Butyl., tert-Butyl, n-Pentyl, Isopentyl-, Neopentyl, n-Hexyl, 2-Methylpentyl, 3-Methylpentyl, 2,2-Dimethylbutyl, 2,3-Dimethylbutyl, n-Heptyl, 2-Methylhexyl, 3-Methylhexyl, 2,2-Dimethylpentyl, 2,3-Dimethylpentyl, 2,4-Dimethylpentyl-, 3,3-Dimethylpentyl, 3-Äthylpentyl, 2,2,3- Trimethylbutyl-, n-Octyl-, 2-Methylhepyl, 3-Methylhepyl, 4-Methylhepyl-, 2,2-Dimethylhexyl-, 2,3-Dimethylhexyl-, 2,4-Dimethylhexyl-, 3,3-Dimethylhexyl-, 3,4-Dimethylhexyl, 4,4-Dimethylhexyl-, 2-Äthylhexyl, 3-Äthylhexyl-, 4-Äthylhexyl-, 2,2,3-Trimethylpentyl-, 2,3,3-Trimethylpentyl, usw. sowie deren cyclische Äquivalente zu verstehen.

Unter C2-C5 Alkenyl sind u.a. Ethen, Propen, 1-Buten, (cis/trans)-2-Buten, 2-Methylpropen, 1-Penten, 2-Penten, 2-Methyl-1-buten, 3-Methyl-1-buten und 2-Methyl-2-buten sowie deren cyclische Äquivalente zu verstehen.

Unter C2-C8 Alkinyl sind u.a. Acetylen, Propin, 1-Butin, 2- Butin, 1-Pentin, 2-Pentin, 3-Methyl-1-butin-, 1-Hexin, 2-Hexin, 3-Hexin, 3,3-Dimethyl-1-butin, 1-Heptin, 2-Heptin, 3-Heptin, 1-Octin, 2-Octin, 3-Octin und 4-Octin sowie deren cyclische Äquivalente zu verstehen.

Unter C1-C4 Alkoxy sind Methoxy, Ethoxy, Propoxy, Isopropoxy, Butoxy, Isobutoxy, sek-Butoxy- und tert-Butoxy zu verstehen.

Unter C6-C12 Aryl sind Phenyl-, Tolyl-, Xylyl-, Naphthyl-, Biphenyl-Ringsysteme zu verstehen.

Je nach den verwendeten Ausgangsverbindungen liegen die zur Herstellung der Monomere benötigten Temperaturen bzw. Drücke zwischen 500 und 1000 °C und kleiner 500 Pa.

Insbesondere sind Endoprothesen mit diesen Eigenschaften für den Einsatz in menschlichen oder tierischen Gefäßen, Gefäßbypässen, Gefäßclips, Filter, Verschlußsysteme, (ummantelten) Stents, Stent-grafts, Urethem, intrahepatischen Bypässen, sowie für den Einsatz in Gallenwegen oder sonstigen Hohlorganen geeignet. Diese können aus Grundmaterial sein ausgewählt aus Metall, Metalllegierungen, Keramik oder Polymeren (z.B. Polyester, Polyamid, Polyurethan, Polyethylen, Polytetrafluoroethylen (PTFE)).

Auf dieser derart erzeugte Oberfläche lassen sich nunmehr erfindungsgemäß bioaktive wasserunlösliche oder in Wasser nur schwer lösliche Wirkstoffe abscheiden. Wasserunlöslich beschreibt in diesem Zusammenhang Substanzen mit einer Löslichkeit von weniger als 0,1 mg/ml in destilliertem Wasser bei 25°C und schwer lösliche Wirkstoffe umfassen Substanzen mit einer Löslichkeit von 0,1 bis 0,9 mg/ml destilliertem Wasser. Als Substanzen kommen hierbei in Betracht: Tretinoin und Tretinoinderivate, Orphanrezeptoragonisten, Elafinderivaten, Corticosteroide und Steroidhormone (wie z.B. Methylprednisolon, Dexamethason, Östradiol), Taxol, Taxolderivate, Rapamune, Tacrolimus, hydrophobe Proteine oder zellproliferationsverändernde Substanzen, oder andere in Wasser nicht oder schwer lösliche zellproliferationsverändernde Substanzen. Die Polymer-beschichtete Endoprothese wird zunächst mit einer Lösung des Wirkstoffes in einem mit Wasser mischbaren Lösungsmittel, wie z.B. Dimethylsulfoxid (DMSO), Dioxan, Dimethylformamid (DMF) oder Tetrahydrofuran (THF) durch Eintauchen, Besprühen oder Pipettieren benetzt. Anschließend wird die Endoprothese in Wasser getaucht, wobei der in Wasser unlösliche Wirkstoff ausfällt, sich teilweise auf der Oberfläche abscheidet und sich partiell in die Polymerschicht einlagert. Das Lösungsmittel wird von der Endoprothese während des Abscheidungsvorgangs abgelöst. Im Rahmen des beschriebenen Verfahren kann sowohl ein einzelner Wirkstoff als auch eine Kombination an Wirkstoffen auf die Endoprothese abgeschieden werden.

Die der Erfindung eigene Abscheidungsmethode unterscheidet sich von anderen Substanzbeschichtungen zum einen durch das Ergebnis der langsamen und gleichmäßigen Wirkstoffabgabe. Zum anderen sind im Zusammenhang mit beschichteten Endoprothesen bisher beschriebene Verfahren Sprüh-, Eintauch-, Pipettierungs- und Luftstromverfahren sowie das Mischen der Substanz mit der aufzutragenden Polymersubstanz (WO 00/32255, WO 00/62830, WO 98/36784). Während bei diesen Verfahren die Dauer des durchgeführten Vorganges für das Ausmaß der Wirkstoffbeladung entscheidend ist, hängt beim vorgeschlagenen Abscheidungsverfahren die aufgebracht Wirkstoffmenge von der Konzentration des Wirkstoffes in der Lösung ab. Des weiteren wurde bisher vorgeschlagen den Wirkstoff mit einem Polymer zu vermischen und diesen dann durch Abbau des Polymers freizusetzen (WO 99/21908). Die Schwierigkeit hydrophobe Substanzen aufzubringen wurde in einem anderen Fall durch die Bildung von Mizellen gelöst, welche dann wiederum aufgetragen wurden (WO 02/085337). Eine weitere Art der Substanzbeschichtung beschreibt die Verwendung negativ geladener therapeutischer Wirkstoffe und kationischer Polyelektrolyte (WO 01/49338). Die Vielzahl der verschiedenen Verfahren und die technische Komplexität der jeweiligen Verfahren macht die Schwierigkeiten deutlich, ein technisch einfaches und verlässliches Verfahren zur Medikamenteabgabe zu entwickeln. Von sämtlichen der bisher beschriebenen Verfahren unterscheidet sich das im Rahmen dieses Patents beschriebene Abscheidungsverfahren.

Das Aufbringen eines Polymers auf eine Endoprothese entspricht dem aktuellen Wissensstand (DE 196 04 173 A1). Ebenso sind bereits verschiedene Verfahren zum Aufbringen eines Wirkstoffes auf eine Polymerschicht beschrieben worden. Hierbei besteht eine der größten Schwierigkeiten in der langsamen Wirkstoffabgabe. Diese wurde versucht dadurch zu lösen, dass eine zweite poröse Polymerbeschichtung auf die erste aufgebracht wird oder aber die erste Polymerbeschichtung die Abgabe des Wirkstoffes beeinflusst (WO 00/32255, WO 98/36784). Im Falle der vorliegenden Erfindung muss nur eine Polymerbeschichtung aufgebracht werden und die Abgabekurve des Wirkstoffes wird auch nicht durch das Polymer beeinflusst, wie bei anderen Drug-Eluting Stents sondern hängt hierbei weitgehend von der Löslichkeit der Wirkstoffinoleküle im Gewebe ab. Im Gegensatz zu anderen Medikamentenabgabe-Systemen lässt sich dadurch ein nahezu konstante Wirkstoffabgabekurve erzielen. Dadurch wird ein gleichmäßige und langdauemde Wirkung erreicht Fig (1). Ein weiterer Vorteil der beschriebenen Methode liegt darin, dass nach dem Abscheiden des Wirkstoffes, nicht unbedingt weitere Beschichtungen aufgebracht werden müssen. Die fehlende Zerstörung des Wirkstoffes während des Aufbringens einer zweiten Polymerschicht ist als besonderes Problem beschrieben worden (WO 98/36784).

Ein minimaler Rest des Wirkstoffes lagert sich bei der beschriebenen Abscheidungsmethode zusätzlich in die Polymerschicht ein und erzielt einen Langzeiteffiekt.

Zur verbesserten Haftung und zur Vergrößerung der Oberfläche können die Außenseite des Stents sowie ggf. auch die Innenseite eine Oberflächenstrukturierung bzw. -profilierung aufweisen. Diese Strukturierung kann aus kleinen Vertiefungen oder Profilierungen bestehen, die die Haftung bzw. Aufnahmefähigkeit der Wirkstoffe verbessern. Eine Strukturierung auf der Außenseite des Stents kann mechanisch, thermisch oder chemisch erreicht werden. Eine Profilierung auf der Innenseite kann in gleicher Weise erfolgen. Hierbei handelt es sich zum Beispiel um kleine Vertiefungen auf der Außenseite des Stents mit einer Tiefe von 5-50 µm und einer Breite von 5-50 µm.

Hinsichtlich des Auftragens eines oder mehrer der oben genannten Wirkstoffe auf der Stentoberfläche bieten sich hierzu folgende Verfahren an:

Die Wirkstoffabscheidung findet auf einer glatten bzw. strukturierten Stentoberfläche statt, die mit einem Polymer - wie z.B. Polyamino p-xylylen-co-polyxylylen - funktionell beschichtet ist.

Die Erfindung wird weiter anhand der beigefügten Figuren beschrieben:

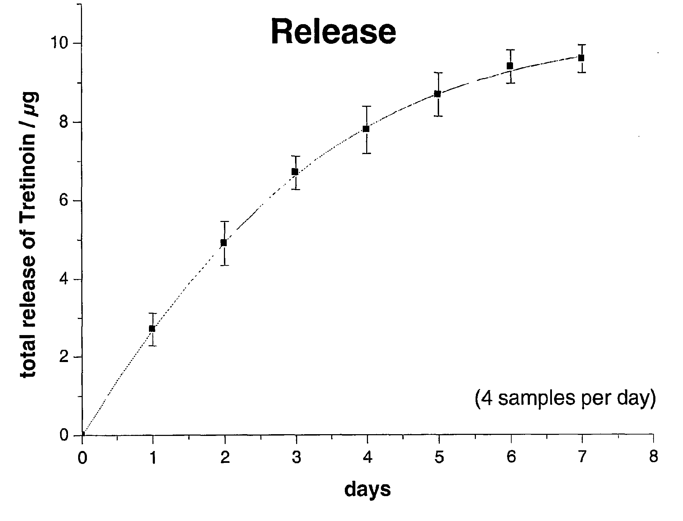

Fig. 1 beschreibt die Abgabekurve von Tretinoin , welches mittels Abscheidungsverfahren auf eine polymerbeschichtete Stentoberfläche aufgebracht wurde; Abgabemedium PBS-Puffer (pH 7,4).

Zur weiteren Erläuterung der Erfindung dienen die nachfolgenden Ausfuhrungsbeispiele:

### Beispiel 1 :

Zur Beschichtung einer Endoprothese wird das Dimere 4-Amino-[2,2]-paracyclophan bei 700°C und 20 Pa in reaktive Monomere gespalten und polymerisiert anschießend auf der auf etwa 20°C gekühlten Stentoberfläche. Die angestrebte Dicke der Polymerbeschichtung beträgt vorteilhaft 10 bis 1000 nm, noch vorteilhafter 200 bis 400 nm. Die nachfolgende nicht kovalente biologische Beschichtung der Oberfläche erfolgt mit Tretinoin oder Tretinoinderivaten. Der mit einer Lösung des Wirkstoffes in Dimethylsulfoxid (DMSO) benetzte polymer-beschichtete Stent wird in Wasser getaucht, wobei der in Wasser unlösliche Wirkstoff ausfällt, sich teilweise auf der Oberfläche abscheidet und sich partiell in die Polymerschicht einlagert.

## Patentansprüche

1. Verfahren zur Erzeugung von bioaktiven Oberflächen auf Gefaßendoprothesen, wobei die Gegenstände zunächst mit einer funktionalisierten Polymerschicht und anschließend mit einer weiteren wirkstoffhaltigen Schicht versehen werden, **dadurch gekennzeichnet, dass** zur Herstellung der funktionellen Polymerschicht aus den Ausgangsverbindungen der allgemeinen Strukturen (1), (2) und/oder (3) bei erhöhten Temperaturen und reduzierten Drücken im wesentlichen Monomere in der Gasphase erzeugt und diese anschließend durch Abkühlung bei reduzierter Temperatur polymerisiert werden, mit: 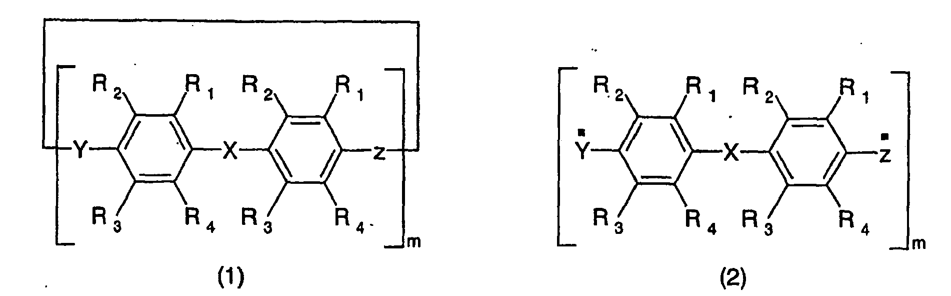 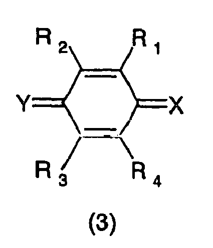 R_{1,2,3,4} : jeweils, gleich oder verschieden voneinander, Wasserstoffatome, Halogenatome, Alkylgruppen bzw. substituierte Alkylgruppen, Arylgruppen bzw. substituierte Arylgruppen, organische Reste oder Radikale, Gruppen der allgemeinen Struktur CO(O-M-A) (mit M: aliphatische oder aromatische Gruppen und A: z.B. Wasserstoff, Hydroxyl-, Amino-, Carboxylgruppen), metallierte Gruppen, Hydroxylgruppen, Aminogruppen, Carboxylgruppen, Estergruppen, Ethergruppen, Säurehalogenidgruppen, Isocyanat- gruppen, schwefelhaltige Gruppen (z.B. Sulfonsäure-, Thioether-, Schwefelsäurerestegruppen), stickstoffltaltige Gruppen (z.B. Nitril-, Amid-, Nitro-, Nitrosamingruppen), phosphorhaltige Gruppen (z.B. Phosphorsäureester-, Phosphonatgruppen), siliziumhaltige Gruppen (z.B. Silyl-, Silyloxygruppen)
X, Y: Kohlenwasserstoffreste: z.B.: Methylen-, Isopropyliden-, Ethylengruppen, funktionalisierte Kohlenwasserstoffreste
m: Zahl der Wiederholungseinheiten =1-20
wobei die zur Herstellung der Monomere benötigten Temperaturen bzw. Drücke zwischen 500 und 1000°C liegen und kleiner 500 Pa sind,
**dadurch gekennzeichnet, dass**
zur Abscheidung der Wirkstoffschicht der mit einer Lösung der/des Wirkstoffe(s) in einem mit Wasser mischbaren Lösungsmittel benetzte polymer-beschichtete Stent in Wasser getaucht wird, wobei der in Wasser unlösliche Wirkstoff ausfallt, sich teilweise auf der Oberfläche abscheidet.

2. Verfahren nach Anspruch 1, **dadurch gekennzeichnet, dass** als mit Wasser mischbares Lösungsmittel Dimethylsulfoxid (DMSO), Dioxan, Dimethylformamid oder Tetrahydrofuran (THF) Verwendung findet.

3. Verfahren nach Anspruch 1 oder 2, **dadurch gekennzeichnet, dass** durch hydrophobe und elektrostatische Wechselwirkungen mit den funktionellen Gruppen der funktionellen Polymerbeschichtung eine höhere Wirkstoffbeladung und Wirkstoffadhäsion im Vergleich zur nicht beschichteten Oberfläche erreicht wird.

4. Verfahren nach Anspruch 1, 2 oder 3, **dadurch gekennzeichnet, dass** sich der/die Wirkstoffe zusätzlich partiell in die Polymerschicht einlagert.

5. Verfahren nach einem der Ansprüche 1 bis 4, **dadurch gekennzeichnet, dass** wasserunlösliche oder in Wasser nur schwer lösliche Wirkstoffe, wie z.B. Tretinoin und Tretinoinderivate, Orphanreceptoragonisten, Elafinderivate, Corticosteroide und Steroidhormone (wie z.B. Methylprednisolon, Dexamethason, Östradiol), Taxol, Taxolderivate, Rapamune, Tacrolimus, hydrophobe Proteine oder zellproliferationsverändernde Substanzen verwendet werden.

6. Verfahren nach einem der Ansprüche 1 bis 5, **dadurch gekennzeichnet, dass** die Kinetik der Wirkstofffreigabe in-vivo von der Gefäßendoprothesenoberfläche durch die Schwerlöslichkeit der/des Wirkstoffes in wässrigen Medien bestimmt wird.

7. Verfahren nach einem der Ansprüche 1 bis 6, **dadurch gekennzeichnet, dass** Dimere der Struktur (1) oder (2) mit m=1 bei Temperaturen zwischen 600 und 900 °C und Drücken kleiner 100 Pa zu Monomeren gespalten werden, und die anschließende Polymerisation bei Temperaturen kleiner 120°C durchgeführt wird.

8. Verfahren nach einem der Anspruche 1 bis 7, **dadurch gekennzeichnet, dass** das auf der Gefäßendoprothese abgeschiedene funktionelle Polymer vorteilhaft eine Schichtdicke zwischen 10 bis 1000 nm aufweist, noch vorteilhafter eine Schichtdicke von 200 - 400 nm.

## Claims

1. A method of producing bioactive surfaces on vessel endoprostheses, wherein the objects are firstly provided with a functionalised polymer layer and then with a further active substance-bearing layer, **characterised in that** substantially monomers are produced in the gaseous phase to produce the functional polymer layer from the starting compounds of the general structures (1), (2) and/or (3) at elevated temperatures and under reduced pressures and they are then polymerised by cooling at reduced temperature, with: 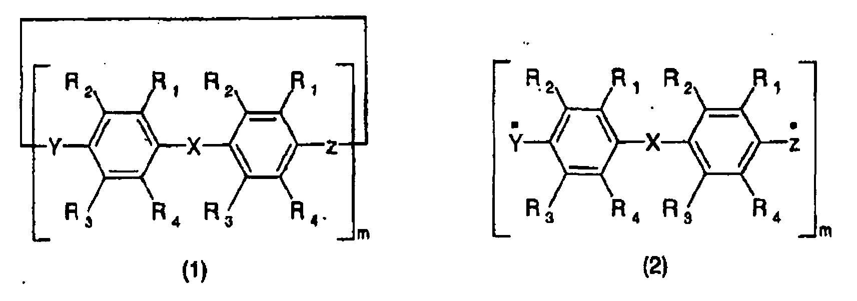 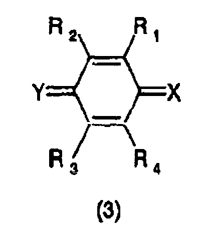 wherein
R_{1,2,3,4} are respectively equal or different from each other hydrogen atoms, halogen atoms, alkyl groups or substituted alkyl groups, aryl groups or substituted aryl groups, organic residues or radicals, groups of the general structure CO(O-M-A) (with M: aliphatic or aromatic groups and A: for example hydrogen, hydroxyl, amino or carboxyl groups), metallated groups, hydroxyl groups, amino groups, carboxyl groups, ester groups, ether groups, acid halide groups, isocyanate groups, sulphur-bearing groups (for example sulphonic acid, thioether and sulphuric acid ester groups), nitrogen-bearing groups (for example nitrile, amide, nitro and nitrosamine groups), phosphorus-bearing groups (for example phosphoric acid ester and phosphonate groups), and silicon-bearing groups (for example silyl and silyloxy groups),
X, Y: hydrocarbon residues: for example: methylene, isopropylidene and ethylene groups, and functionalised hydrocarbon residues; and
m: number of repeating units = 1-20,
wherein the temperatures and pressures required for production of the monomers are between 500 and 1000°C and less than 500 Pa,
**characterised in that**
to deposit the active substance layer the polymer-coated stent wetted with a solution of the active substance or substances in a water-miscible solvent is immersed in water, wherein the active substance which is insoluble in water precipitates and is partially deposited on the surface.

2. A method according to claim 1 **characterised in that** dimethylsulphoxide (DMSO), dioxan, dimethylformamide or tetrahydrofuran (THF) is used as the water-miscible solvent.

3. A method according to claim 1 or claim 2 **characterised in that** a higher active substance loading and active substance adhesion in comparison with the non-coated surface is achieved by hydrophobic and electrostatic interactions with the functional groups of the functional polymer coating.

4. A method according to claim 1, claim 2 or claim 3 **characterised in that** the active substance or substances is additionally partially incorporated into the polymer layer.

5. A method according to one of claims 1 to 4 **characterised in that** water-insoluble active substances or active substances which are only poorly soluble in water such as for example tretinoin and tretinoin derivatives, orphan receptor agonists, elafin derivatives, corticosteroids and steroid hormones (such as for example methylprednisolone, dexamethasone and oestradiol), taxol, taxol derivatives, rapamune, tacrolimus, hydrophobic proteins or cell proliferation-altering substances are used.

6. A method according to one of claims 1 to 5 **characterised in that** the kinetics of active substance release in vivo from the vessel endoprosthesis surface is determined by the poor solubility of the active substance or substances in aqueous media.

7. A method according to one of claims 1 to 6 **characterised in that** dimers of the structure (1) or (2) with m = 1 are cleaved to form monomers at temperatures of between 600 and 900°C and at pressures of less than 100 Pa and subsequent polymerisation is effected at temperatures of less than 120°C.

8. A method according to one of claims 1 to 7 **characterised in that** the functional polymer deposited on the vessel endoprosthesis is advantageously of a layer thickness of between 10 and 1000 nm, still more advantageously a layer thickness of 200 - 400 nm.

## Revendications

1. Procédé de préparation de surfaces bioactives sur des endoprothèses vasculaires, les objets étant d'abord pourvus d'une couche polymère fonctionnalisée puis d'une autre couche contenant un principe actif, **caractérisé en ce que**, pour préparer la couche polymère fonctionnelle à partir des composés de départ ayant les structures générales (1), (2) et/ou (3), on prépare à des températures élevées et sous des pressions réduites pour l'essentiel des monomères en phase gazeuse, puis on les polymérise par refroidissement à une température réduite, avec : 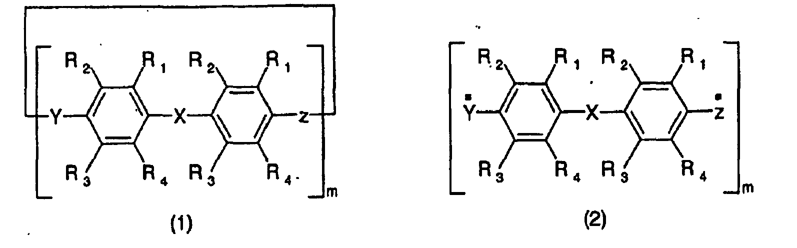 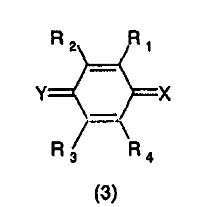 R_{1,2,3,4} sont identiques ou différents les uns des autres, et représentent chacun des atomes d'hydrogène, des atomes d'halogène, des groupes alkyle ou des groupes alkyle substitués, des groupes aryle ou des groupes aryle substitués, des résidus ou radicaux organiques, des groupes de structure générale CO(O-M-A) (où M représente des groupes aliphatiques ou aromatiques et A représente par exemple un atome d'hydrogène ou des groupes hydroxyle, amino, carboxyle), des groupes métallés, des groupes hydroxyle, des groupes amino, des groupes carboxyle, des groupes ester, des groupes éther, des groupes halogénure d'acyle, des groupes isocyanate, des groupes sulfurés (par exemple des groupes acide sulfonique, thioéther, résidus d'acide sulfurique), des groupes azotés (par exemple des groupes nitrile, amide, nitro, nitrosamine), des groupes phosphorés (par exemple des groupes ester d'acide phosphorique, des groupes phosphonate), des groupes contenant du silicium (par exemple des groupes silyle, silyloxy),
X et Y sont des résidus hydrocarbonés, par exemple des groupes méthylène, isopropylidène, éthylène, des résidus hydrocarbonés fonctionnalisés,
m est le nombre des motifs répétitifs et est de 1 à 20,
les températures ou pressions nécessaires à la préparation des monomères étant respectivement comprises entre 500 et 1000°C, et inférieures à 500 Pa,
**caractérisé en ce que**, pour provoquer le dépôt de la couche de principe actif, on immerge dans de l'eau l'endoprothèse, ou stent, revêtue d'un polymère, mouillée par une solution du ou des principes actifs dans un solvant miscible à l'eau, le principe actif insoluble dans l'eau se séparant par précipitation, et se déposant en partie sur la surface.

2. Procédé selon la revendication 1, **caractérisé en ce qu'**on utilise en tant que solvant miscible à l'eau le diméthylsulfoxyde (DMSO), le dioxanne, le diméthylformamide ou le tétrahydrofuranne (THF).

3. Procédé selon la revendication 1 ou 2, **caractérisé en ce que**, grâce à des interactions hydrophobes et électrostatiques avec les groupes fonctionnels du revêtement polymère fonctionnel, on obtient une charge de principe actif et une adhérence du principe actif plus élevées, par comparaison avec une surface non revêtue.

4. Procédé selon la revendication 1, 2 ou 3, **caractérisé en ce que** le/les principes actifs sont en outre partiellement incorporés dans la couche polymère.

5. Procédé selon l'une des revendications 1 à 4, **caractérisé en ce qu'**on utilise des principes actifs insolubles dans l'eau ou seulement difficilement solubles dans l'eau, tels que par exemple la trétinoïne et des dérivés de la trétinoïne, des agonistes des récepteurs orphelins, des dérivés de l'élafine, des corticostéroïdes et des hormones stéroïdiennes (telles par exemple que la méthylprednisolone, la dexaméthasone, l'oestradiol), le taxol, des dérivés du taxol, le Rapamune, le tacrolimus, des protéines hydrophobes ou des substances modifiant la prolifération cellulaire.

6. Procédé selon l'une des revendications 1 à 5, **caractérisé en ce que** la cinétique de la libération du principe actif *in vivo* à partir de la surface des endoprothèses vasculaires est déterminée par l'insolubilité du/des principes actifs dans des milieux aqueux.

7. Procédé selon l'une des revendications 1 à 6, **caractérisé en ce que** des dimères ayant la structure (1) ou (2), dans laquelle m vaut 1, sont dissociés à des températures comprises entre 600 et 900°C et sous des pressions inférieures à 100 Pa pour donner des monomères, et **en ce que** la polymérisation suivante est effectuée à des températures inférieures à 120°C.

8. Procédé selon l'une des revendications 1 à 7, **caractérisé en ce que** le polymère fonctionnel déposé sur l'endoprothèse vasculaire présente avantageusement une épaisseur de couche comprise entre 10 et 1 000 nm et d'une manière encore plus avantageuse une épaisseur de couche de 200 à 400 nm.
